# EUROPEAN PATENT APPLICATION

(11) **EP 0 656 344 A2**
(43) Date of publication of application: **07.06.1995**
(21) Application number: 94307387.4
(22) Date of filing: 07.10.1994
(51) Int. Cl.: C07C 69/80, C07C 69/013, C07C 67/60, C07C 43/196

(54) **Chiral compound and its resolution**

(30) Priority: 15.10.1993 GB 9321343
(71) Applicant: Chiroscience Limited, Cambridge CB4 4WE (GB)
(72) Inventor: McCague, Raymond, Milton, Cambridge CB4 6EE (GB)
(74) Representative: Perry, Robert Edward

(57) **Abstract**

trans-2-Methoxycyclohexanyl hemiphthalate as either single enantiomer, (R) or (S), or as a mixture of enantiomers in which either is in at least 50% ee, is a valuable intermediate in the preparation of resolved 2-methoxycyclohexanol.

## Description

trans-2-Methoxycyclohexanol is a useful synthon; for instance, it can be converted to 2-methoxycyclohexanone which is an intermediate for antibiotic compounds, and potentially as an auxiliary for control of chirality in asymmetric synthesis. For such purposes, it is essential that the trans-2-methoxycyclo-hexanol is used in single-enantiomer form.

trans-2-Methoxycyclohexanol can be resolved by biocatalysis but in the case of a requirement for the [1(S),2(S)]-enantiomer, our experience is that biocatalysis using a commercially-available enzyme such as Amano PS lipase (from Pseudomonas fluorescens) is a lengthy procedure requiring considerable amounts of enzyme.

According to the present invention, an effective procedure has been discovered for the production of enantiomers of 2-methoxycyclohexanol, by resolution of the hemiphthalate ester with a chiral amine. Examples of the chiral amine are the (S) and (R) enantiomers of α-methylbenzylamine (1-phenylethylamine). A schematic representation of the procedure is then as outlined in Scheme 1.

According to the Scheme, racemic trans-methoxycyclohexanol, produced from acid-mediated addition of methanol with cyclohexene oxide, is converted to the hemiphthalate with phthalic anhydride. The (S)-enantiomer of α-methylbenzyl-amine gives crystals that yield the [(S),(S)] enantiomer of 2-methoxycyclohexanol. Typically, the initial crystallisation gives 30% yield of salt, in a diastereo-isomeric excess of 70%. Recrystallisation of the salt elevates the enantiomeric excess. An appropriate solvent is used for the procedure, such as a mixture of ethyl acetate or methyl t-butyl ether with ethanol or 2-propanol.

As an alternative way of operating the process, the enantiomer of the chiral amine can be used that provides the desired enantiomer of the methoxycyclohexanol initially in the liquors. Thus, use of (R)-α-methylbenzylamine may give liquors containing the methoxycyclohexanol component in 70% yield and 30% ee for [(S), (S)] isomer. This enriched material can be broken down to the free hemiphthalate, and then another chiral amine (e.g. (S)-α-methylbenzylamine) used to elevate ee with the benefit of the initial enrichment.

A further feature of the invention is the use of more than one amine (as indicated above), the first to give a partial resolution, and the second to elevate enantiomeric excess. The second amine need not necessarily be chiral, provided that the salt increases in ee on recrystallisation. A corollary of this is that ee elevation can be via a salt of the hemiphthalate with a cationic component that is not an amine, such as an inorganic ion.

The following Example illustrates the invention.

### Example

Racemic 2-methoxycyclohexanyl hemiphthalate (10.0 g) in ethyl acetate (80 ml) was treated with (S)-α-methylbenzylamine (4.84 g) and methanol (3 ml). The mixture was heated until all solid dissolved, then stirred for 24 h at ambient temperature, and lastly maintained at 0°C for 30 min. The crystals were collected by filtration and washed with hexane. This gave the salt in yield of 4.31 g (29%), de 75%.

Recrystallisation from a mixture of ethyl acetate (38 ml) and methanol (3 ml) gave 3.37 g of salt, de 90%.

From a different batch recrystallisation of 2.83 g, 92% de from ethyl acetate (9 ml) and isopropanol (11 ml) gave 2.07 g, de 97%.

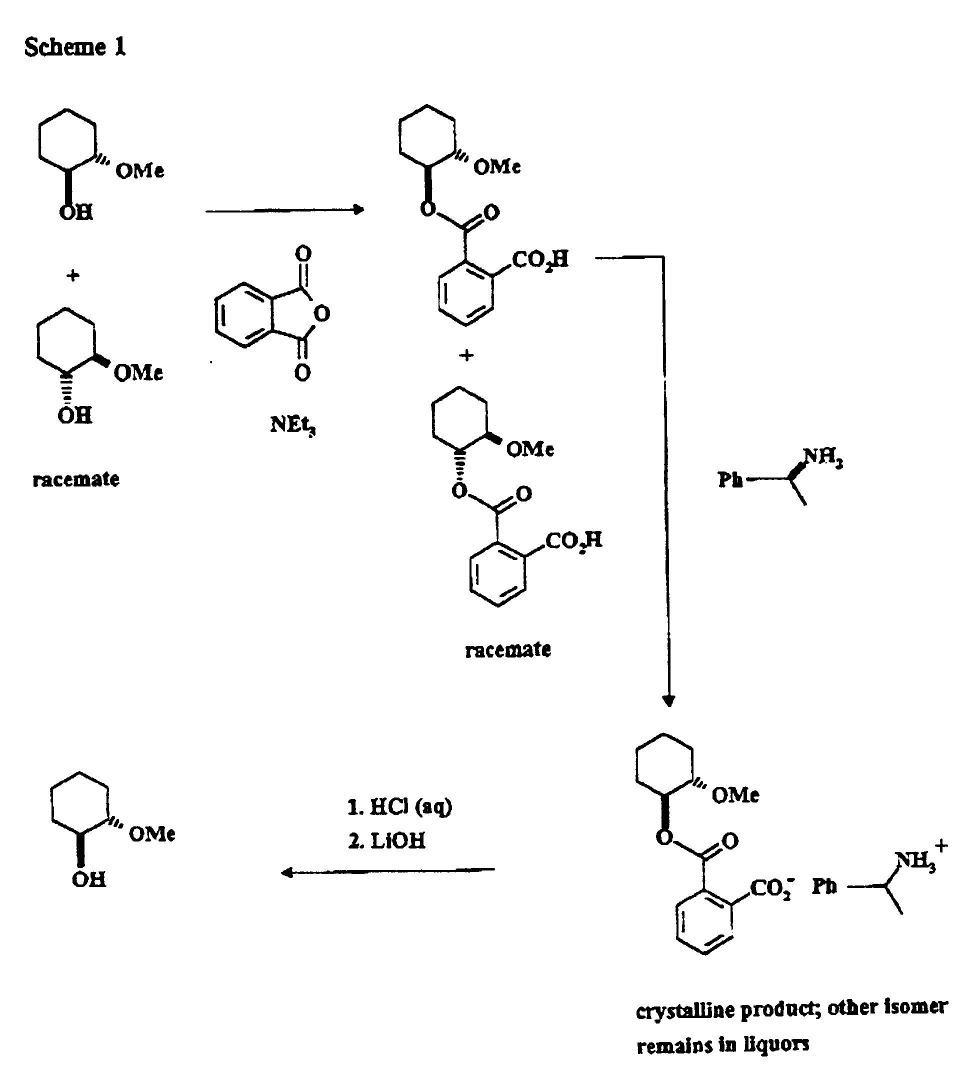

## Claims

1. trans-2-Methoxycyclohexanyl hemiphthalate as either single enantiomer, (R) or (S), or as a mixture of enantiomers in which either is in at least 50% ee.

2. A hemiphthalate according to claim 1, in which the ee is at least 75%.

3. A salt of an amine and a hemiphthalate according to either preceding claim.

4. A salt according to claim 3, in which the amine is optically-active.

5. A salt according to claim 4, which is the (S)-α-methylbenzylamine salt of [1(S), 2(S)]-2-methoxycyclohexanyl hemiphthalate.

6. A salt according to claim 4, which is the (R)-α-methylbenzylamine salt of [1(S) ,2(S)]-2-methoxycyclohexanyl hemiphthalate.

7. Use of a hemiphthalate or salt according to any preceding claim, for the preparation of at least substantially optically-pure 2-methylcyclohexanone.

8. A process for preparing 2-methoxycyclohexanone or trans-2-methoxycyclohexanol as at least substantially a single enantiomer, which comprises converting a mixture of enantiomers of trans-2-methoxycyclohexanol to the hemiphthalate ester; crystallising therefrom a salt according to any of claims 3 to 6 and, if desired, recrystallising the or a different such salt; removing the amine; and, if desired, before or after removing the amine, oxidising the cyclohexanol to the cyclohexanone.
